# EUROPÄISCHE PATENTSCHRIFT

(11) **EP 4 464 346 B1**
(45) Veröffentlichungstag und Bekanntmachung des Hinweises auf die Patenterteilung: **18.02.2026**
(21) Anmeldenummer: 24173829.3
(22) Anmeldetag: 02.05.2024
(51) Int. Cl.: A61M 25/02, A61M 5/158

(54) **FIXIERVORRICHTUNG ZUM FIXIEREN EINES KATHETERS**
FIXATION DEVICE FOR FIXING A CATHETER
DISPOSITIF DE FIXATION POUR FIXER UN CATHÉTER

(30) Priorität: 08.05.2023 DE 102023111956
(43) Veröffentlichungstag der Anmeldung: 20.11.2024
(73) Patentinhaber: B. Braun Melsungen AG, 34212 Melsungen (DE)
(72) Erfinder: Blum, Iris, 34277 Fuldabrück (DE); Berg, Jacqueline, 34212 Melsungen (DE)
(74) Vertreter: Patentanwälte Ruff, Wilhelm, Beier, Dauster & Partner mbB

(56) Entgegenhaltungen:
- GB-A- 2 609 502
- US-A- 5 192 273
- US-A1- 2002 133 121
- US-A1- 2014 276 439

## Beschreibung

Die Erfindung betrifft eine Fixiervorrichtung zum Fixieren eines Katheters auf der Haut eines Patienten mit den oberbegrifflichen Merkmalen des Anspruchs 1.

Katheter weisen üblicherweise einen zwischen einem proximalen Schlauchende und einem distalen Schlauchende längserstreckten biegsamen Katheterschlauch und einen an dem proximalen Schlauchende befestigten Katheteransatz auf, der oftmals auch als Katheterhub (englisch: catheter hub) bezeichnet wird.

Zum Anlegen des Katheters wird der Katheterschlauch auf bekannte Weise, beispielsweise mittels der sogenannten Seldinger-Technik, mit dem distalen Schlauchende voran durch eine Punktionsstelle in der Haut in den Körper des Patienten eingeführt. Der dabei außerhalb des Patienten verbleibende Katheteransatz dient zur fluidleitenden Verbindung des Katheterschlauchs mit weiteren fluidführenden Komponenten, beispielsweise einer Katheterzuleitung oder dergleichen. Auch der Katheterschlauch wird üblicherweise nicht über seine gesamte Länge in den Patienten eingeführt, so dass oftmals ein sich distal an den Katheteransatz anschließender Katheterschlauchabschnitt, der auch als freie Katheterlänge bezeichnet werden kann, außerhalb des Patienten verbleibt.

Es ist üblich, Katheter nach dem Anlegen auf der Haut des Patienten zu fixieren. Hierdurch soll einem unbeabsichtigten Verrutschen des Katheters oder gar einem ungewollten Herausziehen des Katheterschlauchs aus der Punktionsstelle und damit einhergehenden Beeinträchtigungen des Patienten entgegengewirkt werden. Zu diesem Zweck sind unterschiedliche Fixiervorrichtungen bekannt.

Beispielsweise sind Fixiervorrichtungen in Form von Klebepads bekannt, die auf die Haut aufgeklebt werden können und zur Befestigung eines Katheterfilters des zu fixierenden Katheters eingerichtet sind. Weder der Katheteransatz noch der Katheterschlauch des Katheters werden hierbei unmittelbar fixiert. Solche Fixiervorrichtungen werden derzeit unter anderem seitens der Fa. B. Braun Melsungen unter der Bezeichnung Perifix^{®} PinPad oder seitens der Fa. Pajunk unter der Bezeichnung FixoLong am Markt angeboten.

Weiter sind Fixiervorrichtungen bekannt, die ebenfalls auf die Haut aufgeklebt werden können und zur Befestigung des Katheteransatzes des zu fixierenden Katheters eingerichtet sind. Auch diesen Fixiervorrichtungen fehlt es an einer Fixierung des sich distal an den Katheteransatz anschließenden Katheterschlauchs. Solche Fixiervorrichtungen werden derzeit unter anderem seitens der Fa. Pajunk unter der Bezeichnung FixoCath oder seitens der Fa. Bard Medical unter der Bezeichnung StatLock^{®} am Mark angeboten.

Aus der US 2014/276439 A1 ist eine Fixiervorrichtung mit den oberbegrifflichen Merkmalen des Anspruchs 1 bekannt.

Aus der US 2002/133121 A1 ist eine Fixiervorrichtung mit einem Grundkörper und einem Fixierelement zum Fixieren eines Katheteransatzes bekannt.

Aus der US 5 192 273 A ist eine weitere Fixiervorrichtung mit einem Grundkörper und einem Fixierelement zum Fixieren eines Katheteransatzes bekannt.

Aus der GB 2 609 502 A ist eine Vorrichtung zum Sichern von medizinischen Schläuchen bekannt, die einen Grundkörper mit einem Kanal aufweist. Der Kanal ist mit einer Reihe elastisch nachgiebiger Finnen versehen, zwischen denen der zu sichernde Schlauch gehalten werden kann.

Aufgabe der Erfindung ist es, eine Fixiervorrichtung der eingangs genannten Art bereitzustellen, die eine verbesserte Patientensicherheit ermöglicht.

Diese Aufgabe wird durch eine Fixiervorrichtung mit den Merkmalen des Anspruchs 1 gelöst. Vorteilhafte Ausgestaltungen der Erfindung sind Gegenstand der Unteransprüche, deren Wortlaut durch Bezugnahme zum Bestandteil der Beschreibung gemacht wird.

Die erfindungsgemäße Fixiervorrichtung weist einen Grundkörper, ein Fixierelement und eine Führungsaussparung auf. Der Grundkörper weist eine proximodistal erstreckte Längsachse, eine mediolateral erstreckte Querachse und eine anteroposterior erstreckte Hochachse sowie eine posteriore Unterseite auf. Die posteriore Unterseite ist zum wenigstens mittelbaren Auflegen auf die Haut des Patienten eingerichtet. Das Fixierelement ist an dem Grundkörper angeordnet und zum form- und/oder kraftschlüssigen Fixieren eines Katheteransatzes des Katheters eingerichtet. Mittels des Fixierelements ist der Katheteransatz relativ zu dem Grundkörper fixierbar und/oder befestigbar. Die Führungsaussparung ist relativ zu dem Fixierelement distal an dem Grundkörper angeordnet und über eine Führungslänge proximodistal längserstreckt. Die Führungsaussparung weist einander mediolateral gegenüberliegende Führungswandungen auf. Die Führungsaussparung ist zur Aufnahme eines distal an dem Katheteransatz befestigten längserstreckten Katheterschlauchabschnitts eines Katheterschlauchs des Katheters eingerichtet. Der Katheterschlauchabschnitt ist zwischen den Führungswandungen über die Führungslänge führbar und somit gegen ein mediolaterales Ausknicken geschützt. Durch die erfindungsgemäße Lösung wird in erster Linie dem besagten seitlichen Ausknicken oder gar Abknicken des Katheterschlauchabschnitts und damit einhergehenden Beeinträchtigungen des Patienten entgegengewirkt. Hierdurch wird eine verbesserte Patientensicherheit ermöglicht. Zudem kann ein verbesserter Patientenkomfort erreicht werden. Zu diesem Zweck ist die entlang der Längsachse längserstreckte Führungsaussparung mit den einander entlang der Querachse gegenüberliegenden Führungswandungen vorhanden. In fixiertem Zustand ist der distal an den Katheteransatz angrenzende Katheterschlauchabschnitt zwischen den besagten Führungswandungen angeordnet. Hierdurch ist der Katheterschlauchabschnitt wenigstens mediolateral und/oder seitlich, insbesondere begrenzt beweglich, zwischen den Führungswandungen gehalten und auf diese Weise gegen ein Aus- oder gar Abknicken geschützt. Das Fixierelement dient zur lösbaren Befestigung des Katheteransatzes an dem Grundkörper. In fixiertem Zustand des Katheters ist der Katheteransatz mittels des Fixierelements relativ zu dem Grundkörper wenigstens anterior, d.h. gegen ein Abheben nach oben, fixiert. Alternativ oder zusätzlich ist der Katheteransatz mediolateral fixiert. Zu diesem Zweck wirkt das Fixierelement lösbar form- und/oder kraftschlüssig mit dem Katheteransatz zusammen. Das Fixierelement ist bei unterschiedlichen Ausgestaltungen unterschiedlich gestaltet, beispielsweise als Klemm-, Rast- und/oder Steckelement. Der Grundkörper kann grundsätzlich jede geeignete Form aufweisen. Der Grundkörper weist die Längsachse, die Querachse und die Hochachse auf. Die besagten Achsen sind selbstverständlich als geometrische Achsen zu verstehen und bilden ein kartesisches Achsensystem. Die posteriore Unterseite des Grundkörpers ist in der Verwendung der Fixiervorrichtung der Haut des Patienten zugewandt. Zwecks Fixierung wird die posteriore Unterseite unmittelbar oder mittelbar auf die Haut aufgelegt. Die Fixierung kann unter Verwendung weiterer Hilfsmittel, wie beispielsweise eines Pflasters, einer Bandage oder dergleichen erfolgen. Alternativ oder zusätzlich ist ein unmittelbares oder mittelbares Aufkleben des Grundkörpers auf die Haut vorgesehen. Es versteht sich, dass der zu fixierende Katheter nicht Bestandteil der Fixiervorrichtung ist. Die Erfindung betrifft jedoch auch eine Anordnung mit einer erfindungsgemäßen Fixiervorrichtung und einem zu fixierenden oder fixierten Katheter gemäß der vorhergehenden Beschreibung.

Die in dieser Beschreibung verwendeten Lage- und Richtungsangaben, wie proximal, distal, proximodistal, medial, lateral, mediolateral, anterior, posterior und anteroposterior dienen einer vereinfachten Beschreibung und beziehen sich auf einen Verwendungszustand der Fixiervorrichtung, in welchem diese gegebenenfalls gemeinsam mit dem Katheter auf der Haut des Patienten angeordnet ist. Dabei meint "distal" in Richtung der Punktionsstelle. "Proximal" meint von der Punktionsstelle wegweisend. "Posterior" meint in Richtung der unterhalb der Fixiervorrichtung angeordneten Haut des Patienten. "Anterior" meint von der Haut wegweisend. "Medial" und "lateral" beziehen sich auf entgegengesetzte Richtungen parallel zur unterhalb der Fixiervorrichtung befindlichen Haut. Alternativ oder zusätzlich bezeichnen die Angaben "proximal" und "distal" zueinander entgegengesetzte Richtungen entlang der Längsachse. Entsprechendes gilt, mutatis mutandis, hinsichtlich der Angaben "medial" und "lateral" und hinsichtlich der Angaben "anterior" und "posterior" in Bezug auf die Hochachse.

Weiter gemäß der Erfindung weisen die Führungswandungen und/oder der Grundkörper proximodistal voneinander beabstandete und über die Führungslänge verteilt angeordnete Längenmarkierungen auf, welche jeweils einen definierten Längenabschnitt der Führungslänge repräsentieren, wobei der Grundkörper wenigstens im Bereich der Längenmarkierungen durchtrennbar eingerichtet ist. Dabei geht die Erfindung von der Überlegung aus, dass der Katheterschlauchabschnitt, d.h. die freie Katheterlänge, unterschiedlich lang sein kann. Durch die vorhandenen Längenmarkierungen und die durchtrennbare Einrichtung des Grundkörpers kann die Führungslänge - und damit die Länge der Führungsaussparung und der Führungswandungen - auf einfache Weise auf die proximodistale Länge des zu führenden Katheterschlauchabschnitts angepasst, insbesondere gekürzt, werden. Die Längenmarkierungen sind vorzugsweise äquidistant verteilt angeordnet. Bei unterschiedlichen Ausgestaltungen sind die Längenmarkierungen unterschiedlich gestaltet, beispielsweise als Einsenkungen, Auswölbungen, Ziffern, sonstige Buchstaben, Zeichen oder dergleichen. Der Grundkörper kann insbesondere durch eine entsprechende geometrische Gestaltung und/oder Werkstoffwahl durchtrennbar eingerichtet sein. Vorzugsweise bilden die Längenmarkierungen gleichsam Solltrennstellen zum definierten Durchtrennen des Grundkörpers. Die Solltrennstellen bilden jeweils eine mechanische Schwächung des Grundkörpers.

In Ausgestaltung der Erfindung weisen die Führungswandungen und/oder der Grundkörper proximodistal voneinander beabstandete und über die Führungslänge verteilt angeordnete Solltrennstellen auf, welche jeweils eine mechanische Schwächung bilden und zum definierten Durchtrennen eingerichtet sind. Auch diese Ausgestaltung der Erfindung geht von der Überlegung aus, dass die freie Katheterlänge je nach Anwendungsfall variieren kann. Mittels der Solltrennstellen ist die Führungslänge auf besonders einfache Weise an die Länge des zu führenden Katheterschlauchabschnitts anpassbar, insbesondere kürzbar. Zu diesem Zweck wird der Grundkörper im Bereich der Solltrennstellen definiert durchtrennt, beispielsweise indem der Grundkörper im Bereich einer der Solltrennstellen eingerissen, eingeschnitten oder auf sonstige Weise geteilt wird. Jede der Solltrennstellen bildet eine mechanische Schwächung. Zu diesem Zweck können die Solltrennstellen jeweils eine lokale Wandstärkenverringerung, eine Einkerbung oder dergleichen bilden. Denkbar und möglich sind zudem Solltrennstellen in Form jeweils einer Perforation, entlang derer der Grundkörper getrennt werden kann. Vorzugsweise bildet jede der Solltrennstellen eine Längenmarkierung, welche jeweils einen definierten Längenabschnitt der Führungslänge repräsentiert.

In weiterer Ausgestaltung der Erfindung sind die Führungswandungen jeweils durch proximodistal voneinander beabstandete Stegelemente mit dazwischenliegenden Lücken gebildet. Die Stegelemente bilden zwei einander mediolateral, d.h. entlang der Querachse, gegenüberliegende Stegreihen aus, wobei jeweils eine der Stegreihen einer der beiden Führungswandungen zugeordnet ist. Die Stegelemente und/oder die dazwischenliegenden Lücken sind vorzugsweise äquidistant voneinander beabstandet. Vorzugsweise sind die Stegelemente und/oder Lücken jeweils an einer anterioren Oberseite des Grundkörpers angeordnet. Die Stegelemente mit den dazwischenliegenden Lücken erlauben insbesondere eine verbesserte Einsehbarkeit des in der Führungsaussparung aufgenommenen Katheterschlauchabschnitts.

In weiterer Ausgestaltung der Erfindung fungieren die zwischen den Stegelementen angeordneten Lücken jeweils als eine der Längenmarkierungen und/oder eine der Solltrennstellen. Hinsichtlich der Eigenschaften und Funktion der Längenmarkierungen und/oder der Solltrennstellen gilt sinngemäß das bereits zu den vorhergehenden Ausgestaltungen Gesagte, auf das zur Vermeidung von Wiederholungen verwiesen und ausdrücklich Bezug genommen wird.

In weiterer Ausgestaltung der Erfindung beträgt die proximodistale Führungslänge zwischen 10 mm und 60 mm, bevorzugt zwischen 40 mm und 50 mm. Es hat sich gezeigt, dass die vorbezeichneten Wertebereiche für die Führungslänge besonders anforderungsgerecht sind, um einem seitlichen Ausknicken des Katheterschlauchabschnitts wirksam entgegenzuwirken. Sofern die Führungswandungen und/oder der Grundkörper mit Sollbruchstellen versehen sind, ist die Führungslänge auf die vorbezeichnete Weise anpassbar. Beispielsweise kann die Führungslänge in diesem Fall von 60 mm auf 10 mm gekürzt werden, wobei selbstverständlich auch Zwischenwerte entsprechend den vorhandenen Längenmarkierungen und/oder Solltrennstellen möglich sind.

In weiterer Ausgestaltung der Erfindung sind das Fixierelement und die Führungsaussparung jeweils an einer anterioren Oberseite des Grundkörpers angeordnet. Die Oberseite liegt der Unterseite entlang der Hochachse gegenüber. In der Verwendung der Fixiervorrichtung ist die Oberseite der Haut des Patienten abgewandt. Durch die Anordnung des Fixierelements und der Führungsaussparung an der Oberseite des Grundkörpers wird eine verbesserte Einsehbarkeit der fixierten Abschnitte des Katheters erreicht. Hierdurch kann eine nicht ordnungsgemäße Fixierung in verbesserter Weise erkannt werden.

In weiterer Ausgestaltung der Erfindung sind das Fixierelement und die Führungsaussparung jeweils anterior offen, wodurch der Katheteransatz und der Katheterschlauchabschnitt jeweils in posteriorer Richtung in die Fixiervorrichtung einbringbar sind. Durch die nach oben offene Gestaltung des Fixierelements kann der Katheteransatz auf einfache und ergonomische Weise entlang der Hochachse in posteriorer Richtung, d.h. von oben nach unten, in das Fixierelement eingebracht werden. Entsprechendes gilt, mutatis mutandis, für die Aufnahme des Katheterschlauchabschnitts in der Führungsaussparung. Zudem erlaubt diese Ausgestaltung eine vorteilhafte Verteilung der beim Einbringen des Katheteransatzes und des Katheterschlauchabschnitts wirkenden Kräfte auf die Haut des Patienten.

In weiterer Ausgestaltung der Erfindung sind das Fixierelement und die Führungsaussparung jeweils posterior offen, wodurch die Fixiervorrichtung in posteriorer Richtung auf den Katheteransatz und den Katheterschlauchabschnitt aufbringbar ist. Durch die entlang der Hochachse nach unten offene Gestaltung der Führungsaussparung kann diese in posteriorer Richtung, d.h. entlang der Hochachse von oben nach unten, auf den Katheterschlauchabschnitt aufgebracht werden. Entsprechendes gilt, mutatis mutandis, für das Fixierelement in Bezug auf den Katheteransatz. Dies ist eine besonders bevorzugte Ausgestaltung der Erfindung. Insbesondere im Unterschied zu der vorhergehenden Ausgestaltung erlaubt die posterior offene Gestaltung der Führungsaussparung und des Fixierelements eine Fixierung unter weitgehender oder auch vollständiger Vermeidung einer Manipulation des bereits angelegten Katheters. Vereinfacht ausgedrückt, kann die Fixiervorrichtung bei dieser Ausgestaltung der Erfindung von oben nach unten auf den bereits angelegten Katheter aufgebracht werden. Bei der vorhergehenden Ausgestaltung muss der bereits angelegte Katheter hierzu im Unterschied zunächst manuell von der Haut angehoben werden. Hiernach kann die Fixiervorrichtung in angehobenem Zustand des Katheters unter denselben geschoben und dieser sodann posterior in die Fixiervorrichtung eingebracht werden.

In weiterer Ausgestaltung der Erfindung weist die Unterseite eine über die gesamte Länge des Grundkörpers proximodistal längserstreckte Aussparung auf, welche den Grundkörper in eine mediale Körperhälfte und eine laterale Körperhälfte teilt und abschnittsweise durch die Führungsaussparung gebildet ist, wobei die beiden Körperhälften wenigstens mittels des Fixierelements, vorzugsweise elastisch beweglich, miteinander verbunden sind. Durch diese Ausgestaltung der Erfindung kann die Fixiervorrichtung in besonders einfacher und ergonomischer Weise in posteriorer Richtung auf den bereits angelegten Katheter aufgebracht werden. Die Aussparung ist entlang der Längsachse längserstreckt und vorzugsweise zwischen der posterioren Unterseite und der anterioren Oberseite des Grundkörpers entlang der Hochachse durchgängig. Dies gilt vorzugsweise auch für die Führungsaussparung, die einen Abschnitt der Aussparung bildet. Die mediale Körperhälfte und die laterale Körperhälfte liegen einander entlang der Querachse gegenüber. Vorzugsweise bildet die längserstreckte Aussparung eine Symmetrielinie. Vorzugsweise ist der Grundkörper spiegelsymmetrisch in Bezug auf die längserstreckte Aussparung. Die mediale Körperhälfte und die laterale Körperhälfte sind wenigstens mittels des Fixierelements miteinander verbunden. Es versteht sich, dass weitere Querverbindungen zwischen den beiden Körperhälften vorhanden sein können. Bei einer bevorzugten Ausgestaltung ist ein die Körperhälften verbindendes Festkörpergelenk vorhanden, das vorzugsweise durch das Fixierelement gebildet ist, so dass die beiden Körperhälften mittels des Festkörpergelenks begrenzt, vorzugsweise elastisch, gelenkbeweglich miteinander verbunden sind. Die relative Beweglichkeit zwischen den Körperhälften erfolgt in diesem Fall um eine gedachte (virtuelle) und parallel zu der längserstreckten Aussparung orientierte Gelenkachse. Bei einer solchen Gestaltung können die beiden Körperhälften zum vereinfachten Aufbringen der Fixiervorrichtung auf den bereits angelegten Katheter voneinander weggeschwenkt werden. Durch die Schwenkbewegung der beiden Körperhälften wird die Führungsaussparung in mediolateraler Richtung verbreitert. Nach dem Aufbringen erfolgt eine entgegengesetzte Schwenkbewegung, so dass der Katheteransatz entsprechend form- und/oder kraftschlüssig mittels des Fixierelements fixiert und der Katheterschlauchabschnitt in der, dann erneut verengten, Führungsaussparung geführt ist.

In weiterer Ausgestaltung der Erfindung ist ein relativ zu dem Fixierelement proximal an dem Grundkörper angeordnetes Anschlagelement vorhanden, mittels dessen wenigstens eine proximale Beweglichkeit des Katheteransatzes und/oder einer proximal an dem Katheteransatz angeschlossenen fluidführenden Komponente des Katheters formschlüssig begrenzbar ist. Das Anschlagelement fungiert als eine Zugentlastung für proximal gerichtete Zugkräfte. Auf diese Weise kann einem unbeabsichtigten Herausziehen des Katheterschlauchs aus der Punktionsstelle in nochmals verbesserter Weise entgegengewirkt werden. Zur Begrenzung der proximalen Beweglichkeit des Katheteransatzes wirkt das Anschlagelement vorzugsweise unmittelbar mit dem Katheteransatz formschlüssig zusammen. Alternativ oder zusätzlich wirkt das Anschlagelement unmittelbar formschlüssig auf die besagte Komponente, so dass die Beweglichkeit des Katheteransatzes mittelbar formschlüssig begrenzt ist.

In weiterer Ausgestaltung der Erfindung ist der Grundkörper aus einem formstabilen Kunststoffmaterial gefertigt. Hierdurch wird insbesondere eine ausreichende mechanische Beanspruchbarkeit erreicht. Trotz der Fertigung aus dem formstabilen Kunststoffmaterial kann der Grundkörper durch eine entsprechende Gestaltgebung, insbesondere eine entsprechende Dimensionierung seiner Wanddicken, in sich formnachgiebig gestaltet sein.

In weiterer Ausgestaltung der Erfindung ist der Grundkörper relativ zu seiner proximodistalen und mediolateralen Ausdehnung entlang der Hochachse dünn entlang der Hochachse und/oder in Form einer proximodistal und mediolateral flächig erstreckten Platte ausgebildet. Durch eine solche dünne und/oder plattenförmige Gestaltung des Grundkörpers liegt dieser in der Verwendung vergleichsweise eng an der Haut des Patienten an. Hierdurch wird ein verbesserter Patientenkomfort erreicht. Zudem wird durch die dünne und/oder plattenförmige Gestaltung vermieden, dass der Grundkörper sich an Kleidungsstücken des Patienten oder dergleichen verfängt und hierdurch unbeabsichtigt bewegt wird. Zudem erlaubt diese Ausgestaltung eine besonders vorteilhafte Verteilung der beim Einbringen des Katheteransatzes und des Katheterschlauchabschnitts wirkenden Kräfte auf die Haut des Patienten. Dies insbesondere dann, wenn das Fixierelement und die Führungsaussparung jeweils anterior offen sind, so dass der Katheteransatz und der Katheterschlauchabschnitt jeweils in posteriorer Richtung in die Fixiervorrichtung einbringbar sind.

In weiterer Ausgestaltung der Erfindung ist ein formnachgiebiger Kissenkörper vorhanden, auf dessen anteriorer Oberseite der Grundkörper fest aufgebracht ist und dessen posteriore Unterseite zum Auflegen auf die Haut eingerichtet ist, insbesondere wobei eine durch die Unterseite des Kissenkörpers gebildete Auflagefläche um eine Faktor 1,2 bis 3,0, bevorzugt 1,5 bis 2,5, größer ist als die Unterseite des Grundkörpers. Der formnachgiebige Kissenkörper ermöglicht einen nochmals verbesserten Patientenkomfort. Zu diesem Zweck ist der Kissenkörper in Bezug auf die Hochachse zwischen der Haut des Patienten und dem Grundkörper angeordnet und formnachgiebig. Der verbesserte Patientenkomfort wird durch die Formnachgiebigkeit des Kissenkörpers erreicht. Bei einer bevorzugten Ausgestaltung ist der Kissenkörper aus einem geschäumten Kunststoffmaterial gefertigt und/oder weichelastisch. Bei dieser Ausgestaltung der Erfindung ist die Unterseite des Grundkörpers in der Verwendung der Fixiervorrichtung lediglich mittelbar auf die Haut aufgelegt. Die eigentliche Auflagefläche der Fixiervorrichtung wird durch die Unterseite des Kissenkörpers gebildet. Diese ist vorzugsweise um einen Faktor innerhalb der vorgenannten Wertebereiche größer als die Unterseite des Grundkörpers. Hierdurch wird eine verminderte Druckbelastung der Haut erreicht. Dies trägt zu einem nochmals verbesserten Patientenkomfort bei.

In weiterer Ausgestaltung der Erfindung ist eine der Unterseite des Grundkörpers zugeordnete Haftbeschichtung vorhanden und zum lösbaren Aufkleben der Fixiervorrichtung auf die Haut eingerichtet. Die Haftbeschichtung kann unmittelbar an der Unterseite des Grundkörpers angeordnet sein. Sofern ein Kissenkörper vorhanden ist, ist die Haftbeschichtung vorzugsweise auf dessen Unterseite angeordnet. In einem Auslieferungszustand der Fixiervorrichtung ist die Haftbeschichtung vorzugsweise mit einer abziehbaren Folie bedeckt. Nach Abziehen der Folie kann die Fixiervorrichtung mit der Haftbeschichtung voran auf die Haut des Patienten aufgebracht und auf diese Weise lösbar aufgeklebt werden. Hierdurch wird eine besonders einfache Fixierung ohne Zuhilfenahme weiterer Hilfsmittel ermöglicht.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Aufsicht eine Ausführungsform einer erfindungsgemäßen Fixiervorrichtung zum Fixieren eines Katheters,
- Fig. 2: die Fixiervorrichtung nach Fig. 1 in einer schematischen Seitenansicht,
- Fig. 3: in schematischer Aufsicht einen mittels der Fixiervorrichtung nach den Fig. 1 und 2 fixierbaren Katheter,
- Fig. 4: in schematischer Aufsicht eine exemplarische Verwendungssituation, in welcher der Katheter nach Fig. 3 mittels der Fixiervorrichtung nach den Fig. 1 und 2 auf der Haut eines Patienten fixiert ist, und
- Fig. 5: in schematischer Aufsicht eine weitere Ausführungsform einer erfindungsgemäßen Fixiervorrichtung.

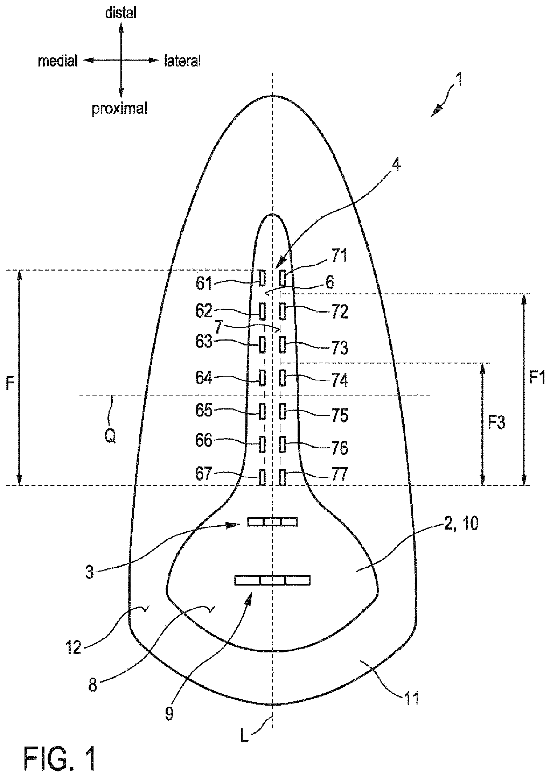

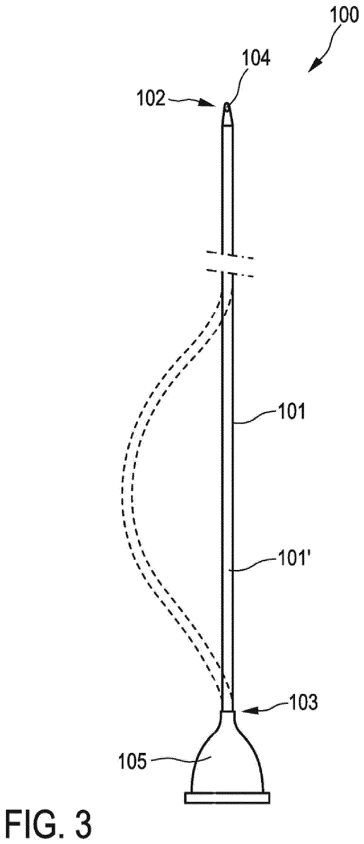

Gemäß den Fig. 1,2 und 4 ist eine Fixiervorrichtung 1 zum Fixieren eines Katheters 100 (Fig. 3) auf der Haut S eines Patienten vorgesehen. Die Fixiervorrichtung 1 eignet sich zur Fixierung üblicher Katheter, wie sie beispielsweise bei einer Infusions-, Transfusions- und/oder Schmerztherapie zum Einsatz kommen.

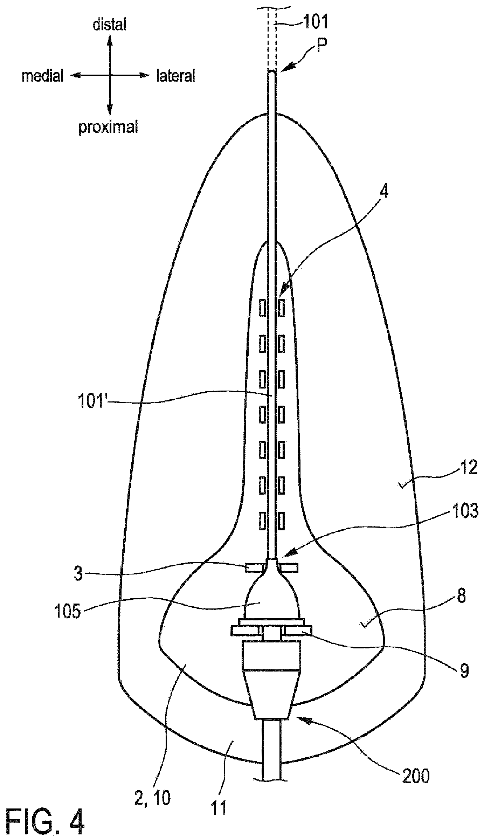

Der exemplarisch in Fig. 3 gezeigte Katheter 100 weist einen dem Fachmann bekannten Aufbau auf. Der Katheter 100 weist einen Katheterschlauch 101 auf, der zwischen einem distalen Katheterschlauchende 102 und einem proximalen Katheterschlauchende 103 längserstreckt ist. Der Katheterschlauch 101 weist ein nicht näher gezeigtes Lumen auf, das im Bereich des distalen Katheterschlauchendes 102 in einen Katheterauslass 104 mündet. Über den Katheterauslass 104 können medizinische Flüssigkeiten aus dem Lumen des Katheterschlauchs 101 abgegeben oder Körperflüssigkeiten oder sonstige Flüssigkeiten in das Lumen aspiriert werden. Der Katheter 100 weist zudem einen Katheteransatz 105 auf. Der Katheteransatz 105 ist fest mit dem proximalen Schlauchende 103 verbunden. Der Katheteransatz 105 dient in erster Linie zur fluidleitenden Verbindung des Katheterschlauchs 101 mit weiteren fluidleitenden Komponenten, beispielsweise einer Katheterzuleitung 200 (siehe Fig. 4) oder dergleichen. Der Katheterschlauch 101 ist aus einem biegsamen Kunststoffmaterial gefertigt. Die hieraus resultierende Deformierbarkeit des Katheterschlauchs 101 ist in Fig. 3 anhand der dort eingezeichneten strichlierten Linien verdeutlicht. Es versteht sich, dass die anhand Fig. 3 gezeigte Gestaltung des Katheters 100 als stark vereinfachend und rein exemplarisch zu verstehen ist.

Die Fixiervorrichtung 1 weist einen Grundkörper 2, ein Fixierelement 3 und eine Führungsaussparung 4 auf.

Der Grundkörper 2 weist eine Längsachse L, eine Querachse Q und eine Hochachse H auf. Die besagten Achsen L, Q, H bilden ein kartesisches Achsensystem und sind orthogonal zueinander ausgerichtet. Die Längsachse L ist proximodistal orientiert. Die Querachse Q ist mediolateral orientiert. Die Hochachse H ist anteroposterior orientiert.

In Bezug auf die anhand Fig. 4 gezeigte Verwendungssituation der Fixiervorrichtung 1 können die besagten Lage- und Orientierungsangaben wie folgt verstanden werden: "Distal" meint in Richtung des distalen Katheterschlauchendes 102 und/oder des Katheterauslasses 104, "proximal" meint von dem distalen Katheterschlauchende 102 weg weisend und/oder in Richtung des proximalen Katheterschlauchendes 103, "posterior" meint der Haut S des Patienten zugewandt, "anterior" meint der Haut S abgewandt, "medial" und "lateral" beziehen sich auf entgegengesetzte Richtungen parallel zu der Haut S des Patienten. Es versteht sich, dass die besagten Lage- und Richtungsangaben für ein vollständiges Verständnis der zugrunde liegenden Erfindung nicht unbedingt notwendig sind, sondern lediglich einer vereinfachten Beschreibung dienen.

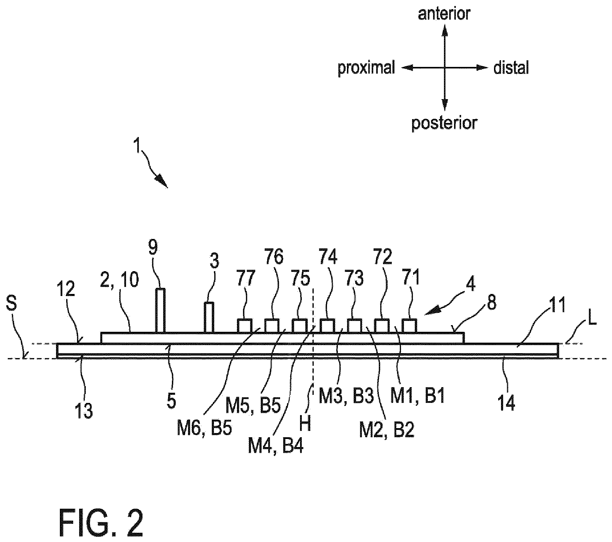

Der Grundkörper 2 weist zudem eine Unterseite 5 auf, die zum wenigstens mittelbaren Auflegen auf die Haut S eingerichtet ist (siehe Fig. 2).

Das Fixierelement 3 ist an dem Grundkörper 2 angeordnet und dient einer form- und/oder kraftschlüssigen Fixierung des Katheteransatzes 105 des zu fixierenden Katheters 100. Das Fixierelement 3 ist dementsprechend eingerichtet und bei der gezeigten Ausführungsform nach Art eines Rastelements gestaltet. Alternativ kann das Fixierelement als Steck- und/oder Klemmelement gestaltet sein. Folglich kann der Katheteransatz 105 zwecks Befestigung an dem Grundkörper mit dem Fixierelement 3 verrastet, verklemmt und/oder zusammengesteckt werden. Dabei sind Details der diesbezüglichen Gestaltung des Fixierelements 3 nicht wesentlich im Hinblick auf die vorliegende Erfindung. Entscheidend ist lediglich, dass das Fixierelement 3 zum form- und/oder kraftschlüssigen Fixieren des Katheteransatzes 105 eingerichtet ist. Das Fixierelement 3 wirkt hierfür unmittelbar mit dem Katheteransatz 105 zusammen und weist beispielsweise eine Rast-, Klemm- und/oder Steckgeometrie auf, die komplementär zu einer Geometrie des Katheteransatzes 105 gestaltet ist. Das Fixierelement 3 ist zur lösbaren Fixierung eingerichtet, so dass der zuvor fixierte Katheteransatz erforderlichenfalls jederzeit erneut von dem Grundkörper 2 gelöst werden kann.

Die Führungsaussparung 4 ist an dem Grundkörper 2 angeordnet und über eine Führungslänge F entlang der Längsachse L längserstreckt. Vorliegend ist die Führungsaussparung 4 parallel zu der Längsachse L. Das Fixierelement 3 ist an einem nicht näher bezeichneten proximalen Ende der Führungsaussparung 4 angeordnet. Die Führungsaussparung 4 weist zwei einander entlang der Querachse Q gegenüberliegende Führungswandungen 6, 7 auf. Die Führungswandungen 6, 7 können vorliegend auch als mediale Führungswandung 6 und laterale Führungswandung 7 bezeichnet werden oder umgekehrt. Die Führungsaussparung 4 ist zur Aufnahme eines Katheterschlauchabschnitts 101' des Katheterschlauchs 101 eingerichtet. Der aufzunehmende Katheterschlauchabschnitt 101' ist ausgehend von dem Katheteransatz 105 und/oder dem proximalen Katheterschlauchende 103 distal längserstreckt. Mit anderen Worten ausgedrückt, grenzt der mittels der Führungsaussparung 4 aufnehmbare Katheterschlauchabschnitt 101' unmittelbar an den Katheteransatz 105 an. Mit nochmals anderen Worten ausgedrückt, handelt es sich bei dem aufzunehmenden Katheterschlauchabschnitt 101'um einen proximalen Abschnitt des Katheterschlauchs 101. Der Katheterschlauchabschnitt 101' ist zwischen den Führungswandungen 6, 7 der Führungsaussparung 4 führbar.

In der anhand Fig. 4 gezeigten Verwendungssituation der Fixiervorrichtung 1 ist der Katheter 100 auf eine dem Fachmann bekannte Weise appliziert und/oder angelegt. Zum Anlegen des Katheters 100 wird der Katheterschlauch 101 mit dem distalen Katheterschlauchende 102 voran durch eine Punktionsstelle P in die Haut S und das darunterliegende Körpergewebe des Patienten eingeführt. Dies beispielsweise mittels der sogenannten Seldinger-Technik. Der Katheteransatz 105 verbleibt außerhalb des Patienten und proximal von der Punktionsstelle P beabstandet. Auch der Katheterschlauch 101 wird nicht vollständig durch die Punktionsstelle P eingeführt, so dass wenigstens der sich unmittelbar distal an den Katheteransatz 105 anschließende Katheterschlauchabschnitt 101' außerhalb des Patienten verbleibt. Der Katheterschlauchabschnitt 101' wird oftmals auch als freie Katheterlänge bezeichnet. Je nachdem, wie weit der Katheterschlauch 101 durch die Punktionsstelle P distal vorgeschoben wird, ändert sich die Länge des Katheterschlauchabschnitts 101'.

In der gezeigten Verwendungssituation der Fixiervorrichtung 1 ist der Grundkörper 2 mit seiner Unterseite 5 voran (wenigstens mittelbar) auf die Haut S aufgelegt und auf noch näher beschriebene Weise lösbar mit dieser verbunden. Der Katheteransatz 105 ist mittels des Fixierelements 3 lösbar an dem Grundkörper 2 befestigt und zu diesem Zweck vorliegend mit dem Fixierelement 3 verrastet. Bei der gezeigten Ausführungsform ist der Katheteransatz 105 in fixiertem Zustand wenigstens entlang der Querachse Q und entlang der Hochachse H relativ zu dem Grundkörper 2 form- und/oder kraftschlüssig gehalten. Zudem ist der Katheteransatz 105 vorliegend entlang der Längsachse L wenigstens in distaler Richtung form- und/oder kraftschlüssig mittels des Fixierelements 3 gehalten.

Weiter ist der Katheterschlauchabschnitt 101' in der Führungsaussparung 4 aufgenommen, zwischen dessen Führungswandungen 6, 7 geführt und folglich wenigstens gegen ein mediolaterales Ausknicken geschützt. Bei der gezeigten Ausführungsform ist ein nicht näher bezeichneter Querabstand zwischen den Führungswandungen 6, 7 derart bemessen, dass der Katheterschlauchabschnitt 101 in seiner radialen Richtung, d.h. entlang der Querachse Q, begrenzt beweglich zwischen den Führungswandungen 6, 7 gehalten ist. Folglich ist der Katheterschlauchabschnitt 101 nicht mittels der Führungswandungen 6, 7 geklemmt oder elastisch deformiert. Bei einer in den Figuren nicht gezeigten Ausführungsform kann der Querabstand zwischen den Führungswandungen derart bemessen sein, dass die Führungsaussparung eine Klemmwirkung auf den Katheterschlauchabschnitt entfaltet.

Bei der gezeigten Ausführungsform beträgt die Führungslänge der Führungsaussparung 4 und damit auch der Führungswandungen 6, 7 50 mm. Bei in den Figuren nicht gezeigten Ausführungsformen beträgt die Führungslänge zwischen 10 mm und 60 mm, bevorzugt zwischen 40 mm und 50 mm.

Bei der gezeigten Ausführungsform sind die Führungswandungen 6, 7 nicht etwa durchgängig und/oder ununterbrochen über die gesamte Führungslänge F längserstreckt. Stattdessen sind die Führungswandungen 6, 7 jeweils durch proximodistal voneinander beabstandete Stegelemente 61 bis 67 und 71 bis 77 gebildet.

Die Stegelemente 61 bis 67 sind der medialen Führungswandung 6 zugeordnet und können daher auch als mediale Stegelemente bezeichnet werden.

Die Stegelemente 71 bis 77 sind der lateralen Führungswandung 7 zugeordnet und können daher auch als laterale Stegelemente bezeichnet werden.

Die Stegelemente 61 bis 67 und 71 bis 77 sind über die Führungslänge F proximodistal äquidistant voneinander beabstandet.

Bei der gezeigten Ausführungsform sind die Stege jeweils 5 mm lang. Bei in den Figuren nicht gezeigten Ausführungsformen sind die Stege jeweils zwischen 5 mm bis 20 mm lang. Die Lücken (ohne Bezugszeichen) zwischen jeweils zwei proximodistal benachbarten Stegelementen sind dementsprechend bemessen. Die Führungswandungen 6, 7 sind zueinander parallel längserstreckt. Entsprechendes gilt folglich auch für die einander gegenüberliegenden medialen Stegelemente 61 bis 67 und die lateralen Stegelemente 71 bis 77.

Bei der gezeigten Ausführungsform fungieren die zwischen den Stegelementen angeordneten Lücken jeweils als eine Längenmarkierung M1 bis M6 und/oder als eine Solltrennstelle B1 bis B6.

Die durch die Lücken gebildeten Längenmarkierungen M1 bis M6 repräsentieren jeweils einen definierten Längenabschnitt der Führungslänge F. Die Längenmarkierungen M1, M2 können auch als erste Längenmarkierung M1 und zweite Längenmarkierung M2 bezeichnet werden. Entsprechendes gilt sinngemäß für die Benennung der weiteren Längenmarkierungen M3 bis M6. Die erste Längenmarkierung M1 repräsentiert eine erste Führungsteillänge F1. Die dritte Längenmarkierung M3 repräsentiert eine dritte Führungsteillänge F3. Entsprechendes gilt sinngemäß für die weiteren Längenmarkierungen. Mit anderen Worten ausgedrückt bilden die Längenmarkierungen M1 bis M6 eine definierte Längeneinteilung für die Führungsaussparung 4 und deren Führungswandungen 6, 7.

Wie oben dargelegt, bilden die Lücken (ohne Bezugszeichen) alternativ oder zusätzlich die Solltrennstellen B1 bis B6 aus. Die Solltrennstellen B1 bis B6 bilden jeweils eine lokale mechanische Schwächung des Grundkörpers 2. Diese lokalen mechanischen Schwächungen erlauben ein definiertes Durchtrennen des Grundkörpers 2 an den besagten Solltrennstellen B1 bis B6. Die mechanische Schwächung kommt vorliegend durch eine Verringerung der Materialstärke des Grundkörpers 2 im Bereich der Lücken zustande. An den Stellen mit jeweils reduzierter Materialstärke, d.h. den Lücken und/oder Solltrennstellen B1 bis B6, kann der Grundkörper 2 daher vergleichsweise einfach durchtrennt werden. Beispielsweise kann der Grundkörper 2 manuell, mittels eines Schneidwerkzeugs oder dergleichen durchtrennt werden. Denkbar und möglich ist auch ein Abreißen. Weiter alternativ oder zusätzlich können die Längenmarkierungen M1 bis M6 und/oder die Solltrennstellen B1 bis B6 jeweils durch eine parallel zur Querachse Q erstreckte Perforation der Grundkörpers 2 gebildet sein. Die Solltrennstellen B1, B2 können auch als erste Solltrennstelle B1 und zweite Solltrennstelle B2 bezeichnet werden. Entsprechendes gilt sinngemäß für die Benennung der weiteren Solltrennstellen B3 bis B6.

Die Längenmarkierungen M1 bis M6 und/oder die Solltrennstellen B1 bis B6 erlauben eine vereinfachte Anpassung, insbesondere Kürzung, der Führungslänge an die tatsächliche freie Länge des Katheterschlauchs 101. Sofern der Katheterschlauch 101 im Vergleich zu der in Fig. 4 gezeigten Verwendungssituation weiter distal durch die Punktionsstelle P vorgeschoben sein sollte, kann die Führungslänge auf einfache Weise gekürzt werden, indem der Grundkörper 2 an einer hierfür passenden Stelle durchtrennt wird. Beispielsweise kann die Führungslänge F auf die Führungsteillänge F1 gekürzt werden, indem der Grundkörper 2 an der zwischen den Stegelementen 61 und 62 sowie 71 und 72 ausgebildeten Lücke (Längenmarkierung M1, Solltrennstelle B1) durchtrennt wird. Sofern der Katheterschlauch 101 noch weiter distal vorgeschoben sein sollte, kann der Grundkörper 2 beispielsweise auf die dritte Führungsteillänge F3 eingekürzt werden. Hierzu wird der Grundkörper 2 an der zwischen den Führungsstegen 63, 64 sowie 73, 74 befindlichen Lücke (dritte Längenmarkierung M3, dritte Solltrennstelle B3) definiert durchtrennt werden.

Bei der anhand der Fig. 1, 2 sowie 4 gezeigten Ausführungsform sind die Führungsaussparung 4 und das Fixierelement 3 jeweils anterior offen. Durch die jeweilige anteriore Öffnung können der Katheteransatz 105 und der Katheterschlauchabschnitt 101' jeweils in posteriorer Richtung in die Fixiervorrichtung 1 eingebracht werden. Genauer: Vorliegend wird der Katheteransatz 105 von oben mit dem Fixierelement 3 verrastet und der Katheterschlauchabschnitt 101' wird von oben in die Führungsaussparung 4 eingebracht. Zwecks Fixierung wird der bereits angelegte Katheter 100 anterior und von der Haut S angehoben. Die Fixiervorrichtung 1 wird vorzugsweise seitlich unter den angehobenen Katheter 100 geschoben und auf die Haut S aufgelegt. Hiernach kann der angehobene Katheter 100 abgesenkt und auf die vorbeschriebene Weise in die Fixiervorrichtung 1 eingebracht werden.

Bei der in den Fig. 1, 2 und 4 gezeigten Ausführungsform sind das Fixierelement 3 und die Führungsaussparung 4 jeweils an einer anterioren Oberseite 8 des Grundkörpers 2 angeordnet. Mit anderen Worten ausgedrückt, ragen die Führungswandungen 6, 7 und das Fixierelement 3 jeweils anterior von dem Grundkörper und/oder dessen Oberseite 8 auf.

Vorliegend weist die Fixiervorrichtung 1 zudem ein Anschlagelement 9 auf. Das Anschlagelement 9 ist an dem Grundkörper 2 angeordnet und proximal von dem Fixierelement 3 und/oder der Führungsaussparung 4 beabstandet. Das Anschlagelement 9 ist zum formschlüssigen Begrenzen einer proximalen Beweglichkeit des Katheteransatzes 105 eingerichtet. Mit anderen Worten ausgedrückt, fungiert das Anschlagelement 9 als unmittelbarer oder mittelbarer Anschlag für den Katheteransatz 105. Hierdurch wirkt das Anschlagelement 9 als eine Zugentlastung, die einem proximalen Herausziehen des Katheterschlauchs 100 aus der Punktionsstelle P entgegenwirkt.

Bei der gezeigten Ausführungsform wirkt das Anschlagelement 9 unmittelbar mit einem nicht näher bezeichneten proximalen Ende des Katheteransatzes 105 zusammen. Zur Begrenzung der Beweglichkeit kontaktiert das Anschlagelement 9 das besagte proximale Ende des Katheteransatzes 105. Bei einer in den Figuren nicht gezeigten Ausführungsform wirkt das Anschlagelement stattdessen mit der Katheterzuleitung 200 zusammen (Fig. 4). Das Anschlagelement 9 ist wiederum an der Oberseite 8 des Grundkörpers 2 angeordnet und ragt anterior von derselben auf.

Bei der gezeigten Ausführungsform ist der Grundkörper 2 in Form einer proximodistal und mediolateral flächig erstreckten Platte 10 gestaltet. Die Platte 10 kann auch als Grundplatte bezeichnet werden. Der Grundkörper 2 ist folglich vorliegend entlang der Hochachse H in Relation dünn und weist eine geringe Materialstärke im Vergleich zu seiner flächigen Ausdehnung auf.

Vorliegend ist der Grundkörper 2 länglich entlang der Längsachse L und vergleichsweise schmal entlang der Querachse Q. Zudem liegt eine Symmetrie in Bezug auf die Längsachse L und damit auch auf die Führungsaussparung 4 vor. Dabei versteht sich, dass die in den Figuren gezeigte Formgebung der gesamten Fixiervorrichtung 1, insbesondere des Grundkörpers 2, als rein exemplarisch zu verstehen ist.

Zudem weist die Fixiervorrichtung 1 vorliegend einen formnachgiebigen Kissenkörper 11 auf. Der Kissenkörper 11 weist eine anteriore Oberseite 12 und eine posteriore Unterseite 13 auf. Der Kissenkörper 11 ist in Bezug auf die Hochachse H zwischen dem Grundkörper 2 und der Haut S angeordnet und dient als weichelastische Unterlage zur Verbesserung des Patientenkomforts. Der Grundkörper 2 ist fest mit der Oberseite 12 des Kissenkörpers 11 verbunden. Dessen Unterseite 13 ist zum Auflegen auf die Haut S eingerichtet. Die Unterseite 5 des Grundkörpers 2 liegt daher vorliegend lediglich mittelbar auf der Haut S auf. Der Kissenkörper 11 ist bei der gezeigten Ausführungsform aus einem elastischen Kunststoffmaterial geschäumt. Der Kissenkörper 11 kann daher auch als Schaumkörper oder Schaumunterlage bezeichnet werden. Die Unterseite 13 des Kissenkörpers 11 ist größer als die Unterseite 5 des Grundkörpers 2. Hierdurch wird eine verringerte Flächenbelastung der Haut S erreicht. Dies mindert Druckstellen oder sonstige Irritationen an der Haut S. Bei der gezeigten Ausführungsform ist eine durch die Unterseite 13 des Kissenkörpers 11 gebildete Auflagefläche (ohne Bezugszeichen) um einen Faktor 2,5 größer als die Fläche der Unterseite 5 des Grundkörpers 2. Bei in den Figuren nicht gezeigten Ausführungsformen beträgt dieser Faktor zwischen 1,2 und 3,0.

Zwecks Befestigung ist der Grundkörper 2 vorliegend auf den Kissenkörper 11 aufgeklebt. Grundsätzlich sind aber auch andere Fügetechniken zur Befestigung des Grundkörpers 2 an dem Kissenkörper 11 denkbar und möglich.

Bei der gezeigten Ausführungsform weist die Unterseite 13 des Kissenkörpers 11 zudem eine Haftbeschichtung 14 auf. Die Haftbeschichtung 14 ist zum lösbaren Aufkleben der Fixiervorrichtung 1 auf die Haut S eingerichtet. Die Haftbeschichtung 14 kann aus einem hierfür geeigneten Adhäsivklebstoff gebildet sein. Bei einer in den Figuren nicht gezeigten Ausführungsform weist die Fixiervorrichtung keinen Kissenkörper auf, so dass die Unterseite des Grundkörpers 2 unmittelbar auf der Haut aufliegt. In diesem Fall kann die Unterseite des Grundkörpers 2 mit einer Haftbeschichtung versehen sein. In einem Auslieferungszustand der Fixiervorrichtung 1 ist die Haftbeschichtung 14 mit einer nicht näher gezeigten Folie bedeckt. Die Folie ist abziehbar.

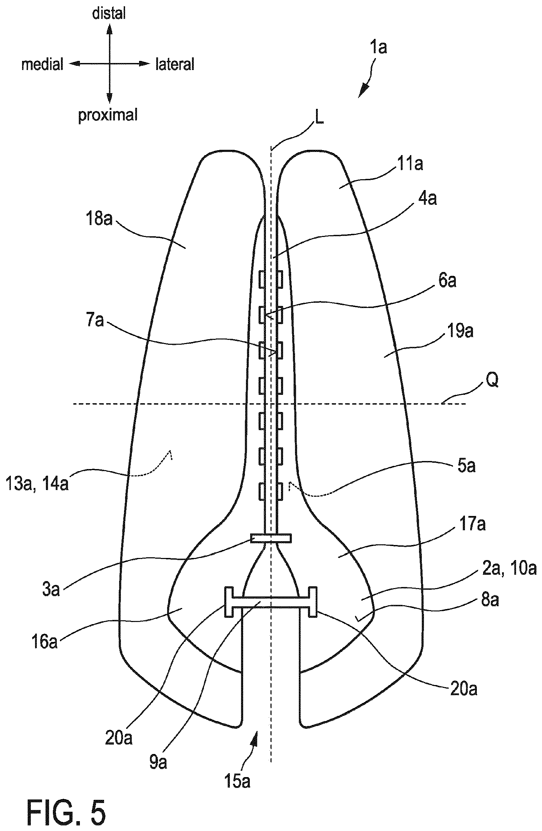

Anhand Fig. 5 ist eine weitere Ausführungsform einer Fixiervorrichtung 1a gezeigt. Die Fixiervorrichtung 1a nach Fig. 5 ist hinsichtlich ihres Aufbaus und ihrer Funktion im Wesentlichen identisch mit der Fixiervorrichtung 1 anhand der Fig. 1, 2 sowie 4. Zur Vermeidung von Wiederholungen wird nachfolgend in erster Linie auf wesentliche Unterschiede der Fixiervorrichtung 1a nach Fig. 5 gegenüber der Fixiervorrichtung 1 nach den Fig. 1, 2 und 4 eingegangen. Im Übrigen gilt das zu der Fixiervorrichtung 1 Offenbarte auch für die Fixiervorrichtung 1a, so dass auf das zu der Ausführungsform nach den Fig. 1, 2 und 4 Gesagte ausdrücklich Bezug genommen und verwiesen wird.

Die Fixiervorrichtung 1a unterscheidet sich im Wesentlichen dadurch von der Fixiervorrichtung 1, dass die Führungsaussparung 4a und das Fixierelement 3a posterior offen sind, so dass die Fixiervorrichtung 1a entlang der Hochachse H von oben nach unten auf den bereits angelegten Katheter 100 aufgebracht werden kann.

Vorliegend weist die Fixiervorrichtung 1a eine proximodistal längserstreckte Aussparung 15a auf, welche den Grundkörper 2a in eine mediale Körperhälfte 16a und eine laterale Körperhälfte 17a teilt. Die Aussparung 15a ist vorliegend über eine gesamte Länge des Grundkörpers 2a längserstreckt. Dabei bildet die Führungsaussparung 4a einen Abschnitt der Aussparung 15a.

Die Fixiervorrichtung 1a ist wiederum mit einem optionalen Kissenkörper 11a versehen. Auch dieser ist mittels der Aussparung 15a in zwei Körperhälften 18a, 19a geteilt. Die Aussparung 15a erstreckt sich vorliegend über eine gesamte Länge des Kissenkörpers 11a.

Die mediale Körperhälfte 16a und die laterale Körperhälfte 17a des Kissenkörpers 11a sind symmetrisch in Bezug auf die Längsachse L und/oder die Aussparung 15a. Entsprechendes gilt sinngemäß für die beiden Körperhälften 18a, 19a des Kissenkörpers 11a.

Die beiden Körperhälften 16a, 17a des Grundkörpers 2a sind vorliegend mittels des Fixierelements 3a miteinander verbunden. Das Fixierelement 3a überbrückt die Führungsaussparung 15a entlang der Querachse Q und ist einends mit der medialen Körperhälfte 16a und andernends mit der lateralen Körperhälfte 17a verbunden.

Die Fixiervorrichtung 1a weist wiederum ein optionales Anschlagelement 9a auf. Vorliegend überbrückt auch das Anschlagelement 9a die Aussparung 15a und bildet eine weitere mechanische Verbindung zwischen den beiden Körperhälften 16a, 17a.

Vorliegend weist das Anschlagelement 9a Griffabschnitte 20a auf, die einer vereinfachten Handhabung der Fixiervorrichtung 1a dienen. Die Griffabschnitte 20a sind entlang der Querachse Q außenliegend an dem Anschlagelement 9a angeordnet und können beispielsweise zwischen dem Daumen und dem Zeigefinger einer Hand gegriffen werden.

Bei der gezeigten Ausführungsform fungieren das Fixierelement 3a und das Anschlagelement 9a gleichsam als gelenkige Verbindung zwischen den beiden Körperhälften 16a, 17a. Mit anderen Worten ausgedrückt, bildet das Fixierelement 3a und/oder das Anschlagelement 9a ein Festkörpergelenk, welches eine um die Längsachse L gerichtete Schwenkbewegung zwischen den beiden Körperhälften 16a, 17a erlaubt.

Durch ein Zusammendrücken der Griffabschnitte 20a zwischen Daumen und Zeigefinger werden die Körperhälften 16a, 17a vorliegend um die Längsachse L und anterior aufeinander zu geschwenkt. Hierdurch werden die Führungswandungen 6a, 7a in Bezug auf die Querachse Q voneinander wegbewegt, was zu einem Aufweiten der Führungsaussparung 4a führt. Hierdurch kann die Fixiervorrichtung 1a in vereinfachter Weise auf den bereits angelegten Katheter 100 aufgebracht werden. Aufgrund einer entsprechenden Materialwahl sind die Gelenkeigenschaften vorliegend elastisch, so dass die Körperhälften 16a, 17a nach einem Lösen der Finger von den Griffabschnitten 20a selbsttätig um die Längsachse L aufeinander zu schwenken.

Bei einer in den Figuren nicht gezeigten Ausführungsform sind das Fixierelement und die Führungsaussparung in Bezug auf die Hochachse beidseits offen. Bei einer solchen Gestaltung kann die Fixiervorrichtung entweder posterior auf den bereits angelegten Katheter aufgebracht werden oder umgekehrt kann der Katheter angehoben und zum Einbringen in die Fixiervorrichtung posterior abgesenkt werden.

## Patentansprüche

1. Fixiervorrichtung (1, 1a) zum Fixieren eines Katheters (100) auf der Haut (S) eines Patienten, aufweisend
einen Grundkörper (2, 2a) mit einer proximodistal erstreckten Längsachse (L), einer mediolateral erstreckten Querachse (Q) und einer anteroposterior erstreckten Hochachse (H) sowie mit einer posterioren Unterseite (5, 5a), die zum Auflegen auf die Haut (S) eingerichtet ist,
ein an dem Grundkörper (2, 2a) angeordnetes Fixierelement (3, 3a), welches zum form- und/oder kraftschlüssigen Fixieren eines Katheteransatzes (105) des Katheters (100) eingerichtet ist und mittels dessen der Katheteransatz (105) relativ zu dem Grundkörper (2, 2a) fixierbar ist, und
eine relativ zu dem Fixierelement (3, 3a) distal an dem Grundkörper (2, 2a) angeordnete sowie über eine Führungslänge (F) proximodistal längserstreckte Führungsaussparung (4, 4a) mit einander mediolateral gegenüberliegenden Führungswandungen (6, 6a, 7, 7a), wobei die Führungsaussparung (4, 4a) zur Aufnahme eines distal an dem Katheteransatz (105) befestigten längserstreckten Katheterschlauchabschnitts (101') eines Katheterschlauchs (101) des Katheters (100) eingerichtet ist, und wobei der Katheterschlauchabschnitt (101') zwischen den Führungswandungen (6, 6a, 7, 7a) über die Führungslänge (F) führbar und somit gegen ein mediolaterales Ausknicken geschützt ist,
**dadurch gekennzeichnet, dass** die Führungswandungen (6, 6a, 7, 7a) und/oder der Grundkörper (2, 2a) proximodistal voneinander beabstandete und über die Führungslänge (F) verteilt angeordnete Längenmarkierungen (M1 bis M6) aufweist, welche jeweils einen definierten Längenabschnitt der Führungslänge (F) repräsentieren, wobei der Grundkörper (2, 2a) wenigstens im Bereich der Längenmarkierungen (M1 bis M6) durchtrennbar eingerichtet ist.

2. Fixiervorrichtung (1, 1a) nach Anspruch 1, **dadurch gekennzeichnet, dass** die Führungswandungen (6, 6a, 7, 7a) und/oder der Grundkörper (2, 2a) proximodistal voneinander beabstandete und über die Führungslänge (F) verteilt angeordnete Solltrennstellen (B1 bis B6) aufweist, welche jeweils eine mechanische Schwächung bilden und zum definierten Durchtrennen eingerichtet sind.

3. Fixiervorrichtung (1, 1a) nach einem der vorhergehenden Ansprüche, **dadurch gekennzeichnet, dass** die Führungswandungen (6, 6a, 7, 7a) jeweils durch proximodistal voneinander beabstandete Stegelemente (61 bis 67, 71 bis 77) mit dazwischenliegenden Lücken gebildet sind.

4. Fixiervorrichtung (1, 1a) nach Anspruch 3 und einem der Ansprüche 1 oder 2, **dadurch gekennzeichnet, dass** die zwischen den Stegelementen (61 bis 67, 71 bis 77) angeordneten Lücken jeweils eine der Längenmarkierungen (M1 bis M6) und/oder eine der Solltrennstellen (B1 bis B6) bilden.

5. Fixiervorrichtung (1, 1a) nach einem der vorhergehenden Ansprüche, **dadurch gekennzeichnet, dass** die proximodistale Führungslänge (F) zwischen 10 mm und 60 mm, bevorzugt zwischen 40 mm und 50 mm, beträgt.

6. Fixiervorrichtung (1, 1a) nach einem der vorhergehenden Ansprüche, **dadurch gekennzeichnet, dass** das Fixierelement (3, 3a) und die Führungsaussparung (4, 4a) jeweils an einer anterioren Oberseite (8, 8a) des Grundkörpers (2, 2a) angeordnet sind.

7. Fixiervorrichtung (1) nach einem der vorhergehenden Ansprüche, **dadurch gekennzeichnet, dass** das Fixierelement (3) und die Führungsaussparung (4) jeweils anterior offen sind, wodurch der Katheteransatz (105) und der Katheterschlauchabschnitt (101') jeweils in posteriorer Richtung in die Fixiervorrichtung (1) einbringbar sind.

8. Fixiervorrichtung (1a) nach einem der vorhergehenden Ansprüche, **dadurch gekennzeichnet, dass** das Fixierelement (3a) und die Führungsaussparung (4a) jeweils posterior offen sind, wodurch die Fixiervorrichtung (1a) in posteriorer Richtung auf den Katheteransatz (105) und den Katheterschlauchabschnitt (101') aufbringbar ist.

9. Fixiervorrichtung (1a) nach Anspruch 8, **dadurch gekennzeichnet, dass** die Unterseite (5a) des Grundkörpers (2a) eine über die gesamte Länge des Grundkörpers (2a) proximodistal längserstreckte Aussparung (15a) aufweist, welche dem Grundkörper (2a) in eine mediale Körperhälfte (16a) und eine laterale Körperhälfte (17a) teilt und abschnittsweise durch die Führungsaussparung (4a) gebildet ist, wobei die beiden Körperhälften (16a, 17a) wenigstens mittels des Fixierelements (3a), vorzugsweise elastisch beweglich, miteinander verbunden sind.

10. Fixiervorrichtung (1, 1a) nach einem der vorhergehenden Ansprüche, **dadurch gekennzeichnet, dass** ein relativ zu dem Fixierelement (3, 3a) proximal an dem Grundkörper (2, 2a) angeordnetes Anschlagelement (9, 9a) vorhanden ist, mittels dessen wenigstens eine proximale Beweglichkeit des Katheteransatzes (105) und/oder einer proximal an dem Katheteransatz (105) angeschlossenen fluidführenden Komponente (200) des Katheters (100) formschlüssig begrenzbar ist.

11. Fixiervorrichtung (1, 1a) nach einem der vorhergehenden Ansprüche, **dadurch gekennzeichnet, dass** der Grundkörper (2, 2a) aus einem formstabilen Kunststoffmaterial gefertigt ist.

12. Fixiervorrichtung (1, 1a) nach einem der vorhergehenden Ansprüche, **dadurch gekennzeichnet, dass** der Grundkörper (2, 2a) relativ zu seiner proximodistalen und seiner mediolateralen Ausdehnung dünn entlang der Hochachse ist und/oder in Form einer proximodistal und mediolateral flächig erstreckten Platte (10, 10a) ausgebildet ist.

13. Fixiervorrichtung (1, 1a) nach einem der vorhergehenden Ansprüche, **dadurch gekennzeichnet, dass** ein formnachgiebiger Kissenkörper (11, 11a) vorhanden ist, auf dessen anteriorer Oberseite (12, 12a) der Grundkörper (2, 2a) fest aufgebracht ist und dessen posteriore Unterseite (13, 13a) zum Auflegen auf die Haut (S) eingerichtet ist, insbesondere wobei eine durch die Unterseite (13, 13a) des Kissenkörpers gebildete Auflagefläche um einen Faktor 1,2 bis 3,0, bevorzugt 1,5 bis 2,5, größer ist als die Unterseite (5, 5a) des Grundkörpers (2, 2a).

14. Fixiervorrichtung (1, 1a) nach einem der vorhergehenden Ansprüche, **dadurch gekennzeichnet, dass** eine der Unterseite (5, 5a) des Grundkörpers (2, 2a) zugeordnete Haftbeschichtung (14, 14a) vorhanden und zum lösbaren Aufkleben der Fixiervorrichtung (1, 1a) auf die Haut (S) eingerichtet ist.

## Claims

1. Fixing device (1, 1a) for fixing a catheter (100) on the skin (S) of a patient, having
a base body (2, 2a) with a proximodistal longitudinal axis (L), a mediolateral transverse axis (Q) and an anteroposterior vertical axis (H), and with a posterior underside (5, 5a) which is configured for placing on the skin (S),
a fixing element (3, 3a) which is arranged on the base body (2, 2a) and which is configured for form-fit and/or force-fit fixing of a catheter hub (105) of the catheter (100) and by means of which the catheter hub (105) is fixable relative to the base body (2, 2a), and
a guide recess (4, 4a) which is arranged distally on the base body (2, 2a) relative to the fixing element (3, 3a) and which extends proximodistally over a guide length (F) and has mediolaterally opposite guide walls (6, 6a, 7, 7a), wherein the guide recess (4, 4a) is configured to receive an elongate catheter tube portion (101'), of a catheter tube (101) of the catheter (100), secured distally to the catheter hub (105), and wherein the catheter tube portion (101') is guidable between the guide walls (6, 6a, 7, 7a) over the guide length (F) and is thus protected against mediolateral buckling,
**characterized in that** the guide walls (6, 6a, 7, 7a) and/or the base body (2, 2a) have/has length markings (M1 to M6) spaced proximodistally apart from one another and distributed over the guide length (F), each representing a defined length portion of the guide length (F), wherein the base body (2, 2a) is able to be severed at least in the region of the length markings (M1 to M6).

2. Fixing device (1, 1a) as claimed in claim 1, **characterized in that** the guide walls (6, 6a, 7, 7a) and/or the base body (2, 2a) have/has predetermined separation points (B1 to B6) spaced proximodistally apart from one another and distributed over the guide length (F), each forming a mechanical weakening and being able to be severed in a defined manner.

3. Fixing device (1, 1a) as claimed in any one of the preceding claims, **characterized in that** the guide walls (6, 6a, 7, 7a) are each formed by proximodistally spaced apart web elements (61 to 67, 71 to 77) with intermediate gaps.

4. Fixing device (1, 1a) as claimed in claim 3 and either of claims 1 and 2, **characterized in that** the gaps arranged between the web elements (61 to 67, 71 to 77) each form one of the length markings (M1 to M6) and/or one of the predetermined separation points (B1 to B6).

5. Fixing device (1, 1a) as claimed in any one of the preceding claims, **characterized in that** the proximodistal guide length (F) measures between 10 mm and 60 mm, preferably between 40 mm and 50 mm.

6. Fixing device (1, 1a) as claimed in any one of the preceding claims, **characterized in that** the fixing element (3, 3a) and the guide recess (4, 4a) are each arranged on an anterior top (8, 8a) of the base body (2, 2a).

7. Fixing device (1) as claimed in any one of the preceding claims, **characterized in that** the fixing element (3) and the guide recess (4) are each open anteriorly, as a result of which the catheter hub (105) and the catheter tube portion (101') are each insertable in the posterior direction into the fixing device (1) .

8. Fixing device (1a) as claimed in any one of the preceding claims, **characterized in that** the fixing element (3a) and the guide recess (4a) are each open posteriorly, as a result of which the fixing device (1a) can be mounted in the posterior direction onto the catheter hub (105) and the catheter tube portion (101') .

9. Fixing device (1a) as claimed in claim 8, **characterized in that** the underside (5a) of the base body (2a) has a recess (15a) which extends proximodistally over the entire length of the base body (2a) and which divides the base body (2a) into a medial body half (16a) and a lateral body half (17a) and is formed in part by the guide recess (4a), wherein the two body halves (16a, 17a) are connected to each other, preferably elastically movably, at least by means of the fixing element (3a).

10. Fixing device (1, 1a) as claimed in any one of the preceding claims, **characterized in that** that a stop element (9, 9a) is present which is arranged proximally on the base body (2, 2a) relative to the fixing element (3, 3a) and by means of which at least a proximal mobility of the catheter hub (105) and/or of a fluid-carrying component (200) of the catheter (100) attached to the catheter hub (105) can be positively limited.

11. Fixing device (1, 1a) as claimed in any one of the preceding claims, **characterized in that** the base body (2, 2a) is made of a dimensionally stable plastics material.

12. Fixing device (1, 1a) as claimed in any one of the preceding claims, **characterized in that** the base body (2, 2a), relative to its proximodistal and its mediolateral extent, is thin along the vertical axis and/or is designed in the form of a proximodistally and mediolaterally planar plate (10, 10a).

13. Fixing device (1, 1a) as claimed in any one of the preceding claims, **characterized in that** a dimensionally flexible cushion body (11, 11a) is present, on the anterior top (12, 12a) of which the base body (2, 2a) is firmly applied, and of which the posterior underside (13, 13a) is configured for placing on the skin (S), in particular wherein a contact surface formed by the underside (13, 13a) of the cushion body is larger by a factor of 1.2 to 3.0, preferably 1.5 to 2.5, than the underside (5, 5a) of the base body (2, 2a).

14. Fixing device (1, 1a) as claimed in any one of the preceding claims, **characterized in that** an adhesive coating (14, 14a) assigned to the underside (5, 5a) of the base body (2, 2a) is present and is configured for releasably gluing the fixing device (1, 1a) to the skin (S).

## Revendications

1. Dispositif de fixation (1, 1a) pour fixer un cathéter (100) sur la peau (S) d'un patient, présentant
un corps de base (2, 2a) avec un axe longitudinal (L) s'étendant de manière proximo-distale, un axe transversal (Q) s'étendant de manière médio-latérale et un axe vertical (H) s'étendant de manière antéro-postérieure, ainsi qu'un côté inférieur postérieur (5, 5a) adapté pour être posé sur la peau (S),
un élément de fixation (3, 3a) agencé sur le corps de base (2, 2a), qui est adapté pour fixer par complémentarité de forme et/ou par adhérence un embout de cathéter (105) du cathéter (100) et au moyen duquel l'embout de cathéter (105) peut être fixé par rapport au corps de base (2, 2a), et
un évidement de guidage (4, 4a) agencé de manière distale par rapport à l'élément de fixation (3, 3a) sur le corps de base (2, 2a) et s'étendant longitudinalement de manière proximo-distale sur une longueur de guidage (F), avec des parois de guidage (6, 6a, 7, 7a) opposées entre elles de manière médio-latérale, l'évidement de guidage (4, 4a) étant adapté pour recevoir une section de tube de cathéter allongée (101') d'un tube de cathéter (101) du cathéter (100), fixée de manière distale sur l'embout de cathéter (105), et la section de tube de cathéter (101') pouvant être guidée entre les parois de guidage (6, 6a, 7, 7a) sur la longueur de guidage (F) et étant ainsi protégée contre un flambage médio-latéral,
**caractérisé en ce que** les parois de guidage (6, 6a, 7, 7a) et/ou le corps de base (2, 2a) présentent des repères de longueur (M1 à M6) espacés de manière proximo-distale les uns des autres et répartis sur la longueur de guidage (F), lesquels représentent chacun une section de longueur définie de la longueur de guidage (F), le corps de base (2, 2a) étant adapté de manière à pouvoir être sectionné au moins dans la zone des repères de longueur (M1 à M6).

2. Dispositif de fixation (1, 1a) selon la revendication 1, **caractérisé en ce que** les parois de guidage (6, 6a, 7, 7a) et/ou le corps de base (2, 2a) présentent des points de séparation théoriques (B1 à B6) espacés de manière proximo-distale les uns des autres et répartis sur la longueur de guidage (F), lesquels forment chacun un affaiblissement mécanique et sont adaptés pour être sectionnés de manière définie.

3. Dispositif de fixation (1, 1a) selon l'une quelconque des revendications précédentes, **caractérisé en ce que** les parois de guidage (6, 6a, 7, 7a) sont formées chacune par des éléments de nervure (61 à 67, 71 à 77) espacés les uns des autres de manière proximo-distale, avec des espaces intermédiaires.

4. Dispositif de fixation (1, 1a) selon la revendication 3 et l'une quelconque des revendications 1 ou 2, **caractérisé en ce que** les espaces agencés entre les éléments de nervure (61 à 67, 71 à 77) forment respectivement l'un des repères de longueur (M1 à M6) et/ou l'un des points de séparation théoriques (B1 à B6).

5. Dispositif de fixation (1, 1a) selon l'une quelconque des revendications précédentes, **caractérisé en ce que** la longueur de guidage proximo-distale (F) est comprise entre 10 mm et 60 mm, de préférence entre 40 mm et 50 mm.

6. Dispositif de fixation (1, 1a) selon l'une quelconque des revendications précédentes, **caractérisé en ce que** l'élément de fixation (3, 3a) et l'évidement de guidage (4, 4a) sont agencés respectivement sur un côté supérieur antérieur (8, 8a) du corps de base (2, 2a).

7. Dispositif de fixation (1) selon l'une quelconque des revendications précédentes, **caractérisé en ce que** l'élément de fixation (3) et l'évidement de guidage (4) sont chacun ouverts antérieurement, moyennant quoi l'embout du cathéter (105) et la section de tube de cathéter (101') peuvent être introduits dans le dispositif de fixation (1) dans la direction postérieure.

8. Dispositif de fixation (1a) selon l'une quelconque des revendications précédentes, **caractérisé en ce que** l'élément de fixation (3a) et l'évidement de guidage (4a) sont chacun ouverts postérieurement, moyennant quoi le dispositif de fixation (1a) peut être appliqué dans la direction postérieure sur l'embout de cathéter (105) et la section de tube de cathéter (101').

9. Dispositif de fixation (1a) selon la revendication 8, **caractérisé en ce que** le côté inférieur (5a) du corps de base (2a) présente un évidement (15a) s'étendant longitudinalement de manière proximo-distale sur toute la longueur du corps de base (2a), lequel divise le corps de base (2a) en une moitié de corps médiale (16a) et une moitié de corps latérale (17a) et est formé par sections par l'évidement de guidage (4a), les deux moitiés de corps (16a, 17a) étant reliées entre elles au moins au moyen de l'élément de fixation (3a), de préférence de manière élastiquement mobile.

10. Dispositif de fixation (1, 1a) selon l'une quelconque des revendications précédentes, **caractérisé en ce qu'**un élément de butée (9, 9a) agencé de manière proximale par rapport à l'élément de fixation (3, 3a) sur le corps de base (2, 2a) est présent, au moyen duquel au moins une mobilité proximale de l'embout de cathéter (105) et/ou d'un composant (200) du cathéter (100) conduisant le fluide raccordé de manière proximale à l'embout de cathéter (105) peut être limitée par complémentarité de forme.

11. Dispositif de fixation (1, 1a) selon l'une quelconque des revendications précédentes, **caractérisé en ce que** le corps de base (2, 2a) est fabriqué à partir d'une matière plastique indéformable.

12. Dispositif de fixation (1, 1a) selon l'une quelconque des revendications précédentes, **caractérisé en ce que** le corps de base (2, 2a) est mince par rapport à son extension proximo-distale et médio-latérale le long de l'axe vertical et/ou est réalisé sous la forme d'une plaque (10, 10a) s'étendant sous forme plate de manière proximo-distale et médio-latérale.

13. Dispositif de fixation (1, 1a) selon l'une quelconque des revendications précédentes, **caractérisé en ce qu'**un corps de coussin souple (11, 11a) est présent, sur le côté supérieur antérieur (12, 12a) duquel le corps de base (2, 2a) est appliqué de manière fixe et dont le côté inférieur postérieur (13, 13a) est adapté pour être posé sur la peau (S), une surface d'appui formée par le côté inférieur (13, 13a) du corps de coussin étant notamment plus grande d'un facteur de 1,2 à 3,0, de préférence de 1,5 à 2,5, que le côté inférieur (5, 5a) du corps de base (2, 2a).

14. Dispositif de fixation (1, 1a) selon l'une quelconque des revendications précédentes, **caractérisé en ce qu'**un revêtement adhésif (14, 14a) associé au côté inférieur (5, 5a) du corps de base (2, 2a) est présent et est adapté pour coller de manière amovible le dispositif de fixation (1, 1a) sur la peau (S).
